# EUROPEAN PATENT APPLICATION

(11) **EP 4 438 070 A1**
(43) Date of publication of application: **02.10.2024**
(21) Application number: 23165339.5
(22) Date of filing: 30.03.2023
(51) Int. Cl.: A61M 1/06

(54) **CONTAINER AND MILK EXPRESSION DEVICE COMPRISING THE SAME**

(71) Applicant: Koninklijke Philips N.V., 5656 AG Eindhoven (NL)
(72) Inventor: CARTEI, Mirko, Eindhoven (NL); JOOSTEN, Franciscus Ivo, Eindhoven (NL); MARSMAN, Albert Willem, Eindhoven (NL); SCHUDELARO, Antonius Adrianus Petrus, Eindhoven (NL)
(74) Representative: Philips Intellectual Property & Standards

(57) **Abstract**

Provided is a container (102) for receiving milk (104) being expressed by a mother. The container is at least partly delimited by a visually transmissive wall (106) arranged to be in sight of the mother while the container is being worn by the mother. Elevation of at least one structural element (108) of, e.g. provided at least partly inside, the container relative to a milk-supporting base (110) of the container enables milk fill level to be estimated by visual inspection of the at least one structural element and milk received in the container. Further provided is a milk expression device (100) comprising the container.

## Description

### FIELD OF THE INVENTION

The invention relates to a container that enables visual estimation of how much expressed breast milk is received therein. The invention further relates to a milk expression device comprising such a container.

### BACKGROUND OF THE INVENTION

Breast pumps and milk collectors are used by breast feeding women to extract milk such that the extracted milk can be fed to their babies at a later time.

With breast pumps, the breast is typically placed into a funnel shaped cup and a vacuum is applied such that milk is extracted. A motorized breast pump typically has two operating modes, namely a stimulation mode and an extraction mode. During the stimulation mode, the milk ejection reflex is stimulated. When the breast pump is activated, a pressure source such as a vacuum pump inside the breast pump generates a vacuum and the stimulation mode can be started. The vacuum pump is typically driven by an electric motor. For portable breast pumps, these motors are driven by battery power. A cyclically applied vacuum causes the mother's milk to be expelled from the nipple of the breast. The milk is then collected in the funnel shaped cup. Gravity usually moves the milk further towards a pipe that is connected to a milk collection container.

There are also breast pumps that use two expression kits (or cups) that may be designed to be worn under a bra, and that are connected to a pump. Such a pump could, for example, be worn on the belt of a user.

Furthermore, there are wearable breast pumps that can be fully worn on the mother's body, allowing the mother to move around freely. Some of these are small enough to fit into a mother's bra. A motor, battery and milk container are, for example, integrated to form a single unit.

There is also a class of cups worn under a bra to which no cyclically applied vacuum is applied. Those devices constitute, for example, simple cups, placed on the breast to collect milk from a breast due to stimulation, for example by a breast pump working on the other breast, or they constitute cups that provide a constant vacuum, for example by virtue of being made, at least in part, of compressible rubber; compression creating a vacuum when the cup is applied to a breast.

### SUMMARY OF THE INVENTION

Whilst there may be advantages to such containers, for example in terms of facilitating movement of the mother during expression, drawbacks associated with monitoring of filling of such containers with milk while they are being worn by the mother expressing milk have been identified.

Conventionally, the mother relies on fill level markings provided on the side of the container. However, such side-positioned fill level markings may be obscured by the mother's bra in which the container is received. The mother tends to be required to stop milk expression and remove the container from the breast, which may involve removing the container from her bra, to check the amount of expressed milk with reference to the side-positioned fill level markings. This interrupting of milk expression is undesirable. Obviously, fill levels can be expressed in different ways, for example as levels relative to a milk-supporting base, volumes, percentages of the total capacity of a container, or a full/not-full indication.

Fill level measurement sensors may be employed to inform the mother of how much milk has been expressed, for example via the mother's smartphone using wireless data transmission from the sensors. However, this approach suffers from being relatively costly, particularly compared to visually estimating milk fill level using fill level markings provided on the side of the container.

The invention is defined by the claims.

According to examples in accordance with an aspect of the invention, there is provided a container for receiving milk being expressed by a mother, the container being at least partly delimited by a visually transmissive wall arranged to be in sight of the mother while the container is being worn by the mother, wherein at least one structural element of the container is arranged, via the at least one structural element's elevation relative to a milk-supporting base of the container, to indicate milk fill level by visual inspection by the mother while wearing the container of the at least one structural element and milk received in the container.

The term "in sight of the mother" is intended to mean that the visually transmissive wall is viewable from a vantage point the same as or similar to that of a user of the container during use of the container, for example viewable between the mother's body and neckline of item(s) of clothing, such as a bra, dress and/or top, in which the container is received when the container is being worn, to enable the mother to perform the visual inspection.

It is noted that this visual inspection is from above the container when the container is being worn by the mother.

The term "in sight of the mother" refers to a direct line of sight to the visually transmissive wall, in other words without optics, e.g. a mirror, being needed to enable the mother to view the visually transmissive wall and perform the visual inspection, for instance when she is wearing the container.

Owing to the arrangement of the visually transmissive wall and the structural element, the container enables the mother to estimate the fill level by visually inspecting the container while she is wearing the container. Accordingly, she is enabled to monitor how much milk is being received in the container during expression without having to pause the expression and remove the container from her breast in order to estimate the fill level by, for instance, inspection of the fill level markings provided on a side wall of the container that is not in the mother's line of sight when wearing the container. The present disclosure also serves to obviate high cost fill level measurement sensor-based solutions.

The at least one structural element can generally be regarded as being provided at least inside the container.

It is noted that the term "container shape element" can, for instance, be used as an alternative to the term "structural element".

The term "elevation relative to a milk-supporting base of the container" refers to height(s) of the structural element(s) relative to the milk-supporting base, which height(s) provide reference(s) against which a level of milk received in the container can be visually compared in order for the fill level to be indicated to the mother.

The at least one structural element may be viewable by the mother looking through the visually transmissive wall.

Implicit in the visually transmissive wall being arranged to be in sight of the mother while the container is being worn by the mother is that the container is wearable, e.g. positionable in the mother's bra, in an orientation that enables the mother to visually inspect the at least one structural element and milk received in the container through the visually transmissive wall. In embodiments this means that at least parts of either or both of the visually transmissive wall and the structural element are not parallel to a mother's line of sight during her visual inspection of the fill level of the container during use of the container.

The term "visually transmissive" as used herein may mean that light is transmittable through the relevant component in order to enable the visual inspection to be performed. The term thus encompasses transparent, e.g. "clear" components, but also translucent components whose visual transmissivity permits the visual inspection to be made.

In some embodiments, the at least one structural element comprises, e.g. is defined by, a visually transmissive element through which milk received in the container is viewable. Thus, the mother may be permitted to view the milk received in the container through the visually transmissive element in order to perform the visual inspection.

The, e.g. each of the, at least one structural element may have a surface contactable by milk when sufficient milk is received in the container to reach the surface.

In such embodiments, the visual inspection may comprise assessing whether or not milk is contacting the surface, or which portion of the surface is being contacted by the milk.

In some embodiments, the surface is included in the visually transmissive element.

In such embodiments, the visually transmissive element may be configured such that light transmitted through the visually transmissive wall is reflected at an interface between the surface and air but refracted at an interface between the surface and milk in contact with the surface.

Different reflection/refraction behavior depending on whether or not milk is contacting the surface, for instance the abovementioned reflection/refraction behavior, may assist the mother to visually discern whether or not the milk is in contact with the surface, or which portion of the surface is being contacted by the milk.

The refractive index of the visually transmissive element, e.g. made of a suitable visually transmissive plastic material, may be selected to be a sufficiently close match with breast milk that light is refracted at the surface-milk interface.

In some embodiments, the surface is provided at a single elevation, in other words level, relative to the milk-supporting base of the container.

In such embodiments, a relatively simple visual estimation of fill level may be enabled, corresponding to a yes/no assessment of whether or not the milk has reached the elevation/level of the surface.

In other embodiments, the surface comprises surface portions arranged at different heights, in other words elevations/levels above the milk-supporting base, of the container indicative of different fill levels of milk received in the container. This may facilitate more detailed monitoring/tracking of filling of the container with milk.

In some embodiments, the surface comprises a continuously sloping surface along which the surface portions are provided. Such a continuously sloping surface may provide a continuous measurement of the fill level.

Alternatively, the surface may comprise a stepped surface that includes one or more riser portions provided between consecutive surface portions. Such a stepped surface may assist to make the visual inspection/fill level estimation relatively robust with respect to tilting of the container.

It is noted that the surface portions and the riser portions may be visually distinguishable from each other with the naked eye. Hence in these embodiments the stepped surface does not effectively constitute a continuously sloping surface by virtue of a possibly large number of surface portions and riser portions.

In some embodiments, the, e.g. each of the, at least one structural element is removable from the container. This removability of the structural element(s) from the container may make the container and the structural element(s) easier to clean and sterilize.

In some embodiments, the visually transmissive element is removable from the container.

In some embodiments, the structural element(s) is or are integrated in the container's wall, e.g. together with the visually transmissive wall.

In such embodiments, no additional manufacturing step may be needed in order to provide the structural element(s), since the structural element(s) is or are manufactured, e.g. molded, along with the rest of the container.

Thus, assembly time may be reduced, e.g. with respect to a scenario in which the structural element(s) is or are distinct component(s) with respect to the container's wall.

In some embodiments, at least part of the structural element(s) is included in the visually transmissive wall. This may offer a relatively straightforwardly way of providing the structural element(s).

In some embodiments, the at least one structural element comprises a ledge portion of the visually transmissive wall. Such a ledge portion may be provided at a defined height or heights relative to the milk-supporting base. Such a ledge portion may be on the inside of the container. Such a ledge portion may be on the outside of the container.

Milk being observed by the mother to align, e.g. make contact, with the ledge portion may be indicative of a given fill level being received in the container.

As an alternative or in addition to the ledge portion of the visually transmissive wall, an exterior surface of the container may have one or more recessed portions that each correspond to a bulge of the container's interior surface, with the bulge being included in the at least one structural element.

In such embodiments, the recessed portion(s) may assist the mother to grip the container, while the corresponding bulge(s) of the container's interior surface may enable the mother to determine fill level by visual inspection of the bulge(s) and the milk received in the container through the visually transmissive wall.

As an alternative or in addition to the ledge portion and/or the recessed portion(s)/bulge(s), the at least one structural element may comprise one or more outwardly protruding portions of the container's exterior surface that each correspond to an indentation in the container's interior surface in which milk is receivable.

In such embodiments, the outwardly protruding portion(s) may assist with locating and/or orientating the container when the container is being donned by the mother. For example, the outwardly protruding portion(s) may assist the mother to locate and/or orientate the container in the mother's bra. Alternatively or additionally, the outwardly protruding portion(s) may assist the mother to grip the container.

Such functionality of the outwardly protruding portion(s) may be in addition to the indentation(s) in the container's interior surface enabling the mother to determine the fill level by visual inspection of the milk received in the indentation(s) through the visually transmissive wall.

In some embodiments, the at least one structural element may comprise one or more indentations in the container's interior surface in which milk is receivable without the at least one structural element comprising one or more corresponding outwardly protruding portions of the container's exterior surface. This may help in providing a container with an external shape desirable for using the container with clothing.

In some embodiments, the at least one structural element may comprise one or more protrusions protruding into the container in which milk is receivable without the at least one structural element comprising one or more corresponding indentations on the container's exterior surface. In such embodiments a user may observe one or more protrusions disappear from view as the fill level increases during milk expression.

The at least one structural element may comprise external shapes of the container, internal shapes of the container, or a combination of both shapes.

In some embodiments, the container comprises fill level markings, such as alphanumeric characters or symbols, paired with the structural element(s). Such fill level markings may, for example, be provided, e.g. embossed, engraved, or printed, on the surface of the visually transmissive element. Alternatively or additionally, numerals indicative of the fill level may be included in the fill level markings. Fill level markings may help a user in qualitatively or quantitatively interpreting the fill level of the container.

In some embodiments, the fill level markings comprise either or both of legible characters and symbols, wherein at least some of these characters and symbols are configured such that they are legible to a mother during use of the container. Depending on the symmetry of the fill level markings, this may mean that the markings are oriented upside-down and left-right reversed compared to a traditional configuration in which these markings are legible not from above, but when the container is viewed from a side.

In some embodiments, the container has lateral left and right sides and comprises an area of maximum circumference measured between the lateral left and right sides, thereby defining superior and inferior sides of the container, and wherein both the superior and inferior sides comprise fill level markings. In some embodiments, the superior side comprises fill level markings oriented upside-down and left-right reversed (because the superior side is the side a mother views from above during use), and the inferior side comprises fill level markings right-side-up, when the container is viewed from a side. In some embodiments, the superior side comprises fill level markings oriented upside-down and left-right reversed (because the superior side is the side a mother views from above during use), and the inferior side comprises fill level markings that are right-side-up, but left-right reversed, when the container is viewed from a side. The left-right reversals allows a mother to read the markings on the inferior side when looking through the container during use of the container.

In some embodiments, fill level markings are laterally displaced relatively to each other. In some embodiments, fill level markings on the superior side are laterally displaced relative to fill level markings on the inferior side. The lateral displacement prevents markings from overlapping when viewed from above as by a user during use of the container.

In some embodiments, the container comprises a background portion, for example a colored portion, for providing a visual contrast with milk that facilitates viewing of the milk. The background portion may also be configured to indicate whether the fill level has reached a predetermined level, for example whether the container is full or not. For example, the background portion may comprise different levels of transparency or one or more colors. One color, for example green, may indicate that the container is not yet full. Another color, for example red, may indicate that the fill level has reached the maximum capacity of the container.

In some embodiments, the container is in the form of a cup receivable in a bra worn by the mother. Such a container in the form of a cup may be receivable, together with the breast on which the cup is worn, in the bra worn by the mother.

According to another aspect there is provided a milk expression device comprising: a funnel for receiving a nipple-comprising portion of a breast, and the container according to any of the embodiments described herein, with the container being for receiving from the funnel milk being expressed by the mother. In some embodiments, the milk expression device is in the form of a cup receivable in a bra worn by the mother. Such a milk expression device in the form of a cup may be receivable, together with the breast on which the cup is worn, in the bra worn by the mother.

The funnel may be included in a first device portion of the milk expression device, with the container being included in a second device portion of the milk expression device.

These first and second device portions can, for example, be regarded as two "halves" of the milk expression device.

In such embodiments, the milk expression device preferably comprises a continuous profile or shape at a point at which the first and second device portions interface with each other.

Such a continuous shape may assist with creating a seal between the first and second device portions.

In some embodiments, the first device portion and the second device portion are configured to be detachably coupled to each other. In other words, the container may be separable from the funnel, for example by the second device portion being separable from the first device portion. This may facilitate cleaning of the funnel and the container.

In some embodiments, the visually transmissive wall is comprised in a flat viewing surface. In some embodiments, this flat viewing surface is configured to be perpendicular to line of sight of a mother during use of the container. Such a flat viewing surface allows easy viewing by the mother of the inside of the container.

In some embodiments, at least one of the visually transmissive wall and the structural element are configured such that they act as a lens. This functionality helps the visual inspection of the fill level by a mother using the container.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 provides a schematic side elevation of a milk expression device according to a first example;
FIG. 2 provides a schematic side elevation of a milk expression device according to a second example;
FIG. 3 provides a schematic side elevation of a milk expression device according to a third example;
FIG. 4 provides a schematic view of a container according to an example as observed by a mother wearing the container;
FIG. 5 provides a schematic view of a container according to another example as observed by a mother wearing the container;
FIG. 6 provides a schematic view of a container according to still another example as observed by a mother wearing the container;
FIG. 7 provides a schematic view of a container according to yet another example as observed by a mother wearing the container;
FIGs. 8A and 8B provide views of a container according to a further example as observed from the side and by a mother wearing the container respectively;
FIGs. 8C and 8D provide the views shown in FIGs. 8A and 8B but with milk partially filling the container;
FIGs. 8E and 8F provide the views shown in FIGs. 8C and 8D but with more milk being received in the container;
FIGs. 9A to 9D provide various schematic views of a container and a visually transmissive element according to examples;
FIG. 10 provides a schematic side elevation of a milk expression device according to a fourth example;
FIG. 11 provides a schematic side elevation of a milk expression device according to a fifth example;
FIG. 12A provides a schematic view of a container defining sides of a container;
FIG. 12B-F provide schematic views of containers according to yet other examples;
FIG. 13 provides a schematic view of a container according to yet another example;
FIG. 14 provides a schematic view of a container according to yet another example; and
FIG. 15 provides a schematic view of a breast pump comprising a container according to yet another example.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a container for receiving milk being expressed by a mother. The container is at least partly delimited by a visually transmissive wall arranged to be in sight of the mother while the container is being worn by the mother. Elevation of at least one structural element of, e.g. provided at least partly inside, the container relative to a milk-supporting base of the container enables the fill level to be estimated by visual inspection of the at least one structural element and milk received in the container. Further provided is a milk expression device comprising the container.

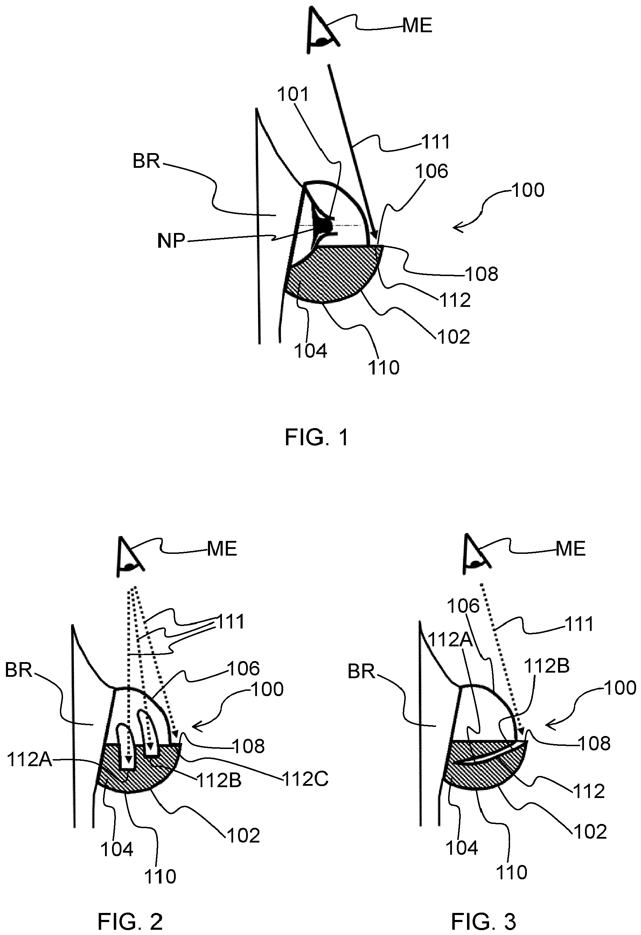

FIG. 1 schematically depicts a milk expression device 100 according to an example. The milk expression device 100 comprises a funnel 101 for receiving a nipple-comprising portion NP of a breast BR. A container 102 receives milk 104 expressed from the nipple of the breast BR. The container 102 may be included, together with the funnel 101, in the milk expression device 100.

A motorized breast pump (not visible) may apply a vacuum that causes the milk 104 to be expressed from the nipple of the breast BR. In such embodiments, for example a cyclically applied vacuum may cause the milk 104 to be expressed from the nipple. The milk 104 is then collected in the container 102.

In some embodiments, the motorized breast pump is a wearable breast pump, for example a wearable breast pump that is wearable in its entirety on the mother's body. In embodiments, a motorized pump is comprised in the milk expression device 100. In other embodiments, a milk expression system comprises the milk expression device 100 and a motorized pump that is external to the milk expression device 100.

A wearable breast pump that is wearable in its entirety on the mother's body may permit the mother to move around freely while expressing milk 104.

In some embodiments, the wearable breast pump is dimensioned to fit into the mother's bra. Alternatively or additionally, a motor and battery of the wearable breast pump are, together with the container 102, integrated to form a single unit.

More generally, the motorized breast pump may be included, together with the funnel 101 and the container 102, in the milk expression device 100.

In alternative embodiments, the container 102 receives milk 104 from the nipple without a vacuum being applied to the breast BR on which the container 102 is worn by a motorized breast pump. Milk 104 may nonetheless be received in the container 102 via suitable stimulation being applied, for instance by milk being extracted from the mother's other breast, e.g. via expression from the mother's other breast using a motorized breast pump.

Alternatively or additionally, a constant vacuum may be applied to the breast BR, for instance by part of the milk expression device 100 being compressible, with compression of the part of the milk expression device 100 creating a vacuum when the compressed part is applied to the breast BR.

Irrespective of the particular technique used to implement and/or stimulate the milk expression, the container 102 is a container 102 for receiving milk 104 being expressed, in other words in the process of being expressed, by the mother.

The container 102 may be worn on one of the mother's breasts BR, as schematically depicted in FIG. 1.

In some embodiments, such as shown in FIG. 1, the container 102 is in the form of a cup receivable in a bra worn by the mother. Such a container 102 in the form of a cup may be receivable, together with the breast BR on which the cup is worn, in the bra worn by the mother.

Whilst there may be advantages to such containers 102, for example in terms of facilitating movement of the mother during expression, drawbacks associated with monitoring of filling of such containers 102 with milk 104 while they are being worn by the mother expressing milk 104 have been identified.

As has been established, via interviews with mothers and user testing, monitoring filling of the container 102 with milk 104, e.g. identifying an amount, e.g. a fill level characterizing the amount of milk that has been expressed, is important for the mother to provide a measure of how successfully milk is being expressed.

Conventionally, the mother relies on fill level markings provided on the side of the container 102, e.g. cup. However, such side-positioned fill level markings may be obscured by the mother's bra in which the container 102 is received. The mother tends to be required to stop milk expression and remove the container 102 from the breast BR, which may involve removing the container 102 from her bra, to check the amount of expressed milk 104 with reference to the side-positioned fill level markings. This interrupting of milk expression is undesirable.

In the case of wearable breast pumps, fill level measurement sensors may be employed to inform the mother of how much milk 104 has been expressed, for example via the mother's smartphone using wireless data transmission from the sensors. However, this approach suffers from being relatively costly, particularly compared to visually estimating milk fill level using fill level markings provided on the side of the container 102.

Correspondingly, and with continued reference to FIG. 1, the container 102 according to the present disclosure is at least partly delimited by a visually transmissive wall 106 arranged to be in sight of the mother while the container 102 is being worn by the mother, with at least one structural element 108 of the container 102 being arranged to indicate the fill level by visual inspection of the at least one structural element 108 and milk 104 received in the container 102. In embodiments, the visual inspection may occur through the visually transmissive wall 106. For example, the visually transmissive wall may be comprised in a wall portion defining an external boundary of a container and the at least one structural element may be an inner surface area of the container, wherein milk may contact the at least one structural element if the milk level reaches the level of the at least one structural element.

The at least one structural element 108 can generally be regarded as being provided at least inside the container 102.

It is noted that the term "container shape element" can, for instance, be used as an alternative to the term "structural element" 108.

Elevation of the at least one structural element 108 of the container 102 relative to a milk-supporting base 110 of the container 102 enables the fill level to be estimated by visual inspection of the at least one structural element 108 and milk 104 received in the container 102 visual.

The term "elevation relative to a milk-supporting base 110 of the container 102" refers to height(s) of the structural element(s) 108 relative to the milk-supporting base 110, which height(s) provide reference(s) against which a level of milk 104 received in the container 102 can be visually compared in order for the fill level to be indicated to the mother.

In embodiments such as the one shown in FIG. 1, the at least one structural element 108 is viewable by the mother looking through the visually transmissive wall 106. Implicit in the visually transmissive wall 106 being arranged to be in sight of the mother while the container 102 is being worn by the mother is that the container 102 is wearable, e.g. positionable in the mother's bra, in an orientation that enables the mother to visually inspect the at least one structural element 108 and milk 104 received in the container 102 through the visually transmissive wall 106. This means that the container must be configured such that the at least one structural element presents a visible aspect to the mother.

The term "in sight of the mother" is intended to mean that the at least one structural element visual is viewable, to enable the mother to perform the visual inspection, from above the container 102 when the container 102 is being worn by the mother. Moreover, the term "in sight of the mother" refers to a direct line of sight 111 to the at least one structural element visual, in other words without optics, e.g. a mirror, being needed to enable the mother to view the visually transmissive wall 106 and perform the visual inspection when she is wearing the container 102.

It is noted that the at least one structural element visual being viewable, to enable the mother to perform the visual inspection, from above the container 102 can be regarded as being related to the mother's breast BR whose milk 104 is receivable in the container 102 being positioned beneath the mother's eyes ME, with the container 102 worn by the mother being correspondingly also positioned beneath the mother's eyes ME.

The term "visually transmissive" as used herein may mean that light is transmittable through the relevant component in order to enable the visual inspection to be performed. The term thus encompasses transparent, e.g. "clear" components, but also translucent components whose visual transmissivity permits the visual inspection to be made.

It is noted that in some embodiments, the container 102 comprises a background portion, for example a colored portion, for providing a visual contrast with milk 104 that facilitates viewing of the milk 104.

The container's 102 wall may be formed from any suitable material. In at least some embodiments, the container's 102 wall is formed from a plastic material, such as polypropylene.

The visually transmissive wall 106 can be formed from a plastic material, such as polypropylene. Plastic material, such as polypropylene, is available having suitable visual transmissivity to enable the user to perform the visual inspection of the at least one structural element 108 and milk 104 received in the container visual.

It is noted that so-called "clear polypropylene" is available whose transparency, among other things, may render this material particularly suitable for the visually transmissive wall 106.

In at least some embodiments, the visually transmissive wall 106 is formed from the same material as the rest of the container's 102 wall.

The container's 102 wall and/or specifically the visually transmissive wall 106 may be formed using any suitable manufacturing process, such as a molding process, e.g. a molding process comprising molding from a plastic material, such as polypropylene.

The structural element(s) 108 may be formed from any suitable material. In some embodiments, the at least one structural element 108 is formed from a plastic material, such as polypropylene, e.g. clear polypropylene.

In some embodiments, the, e.g. each of the, at least one structural element 108 is formed from the same material as that forming the visually transmissive wall 106. This material may be, for example, a plastic material, such as polypropylene, e.g. clear polypropylene.

The structural element(s) 108 may be formed from any suitable manufacturing process, such as a molding process, e.g. a molding process comprising molding the structural element(s) 108 from a plastic material, such as polypropylene.

In some embodiments, such as shown in FIG. 1, at least part of the structural element(s) 108 is included in the visually transmissive wall 106. This may offer a relatively straightforwardly way of providing the structural element(s) 108.

The structural element(s) 108 may, in more general terms, be integrated in the container's 102 wall.

In such embodiments, no additional manufacturing step may be needed in order to provide the structural element(s) 108, since the structural element(s) 108 is or are manufactured, e.g. molded, along with the rest of the container 102.

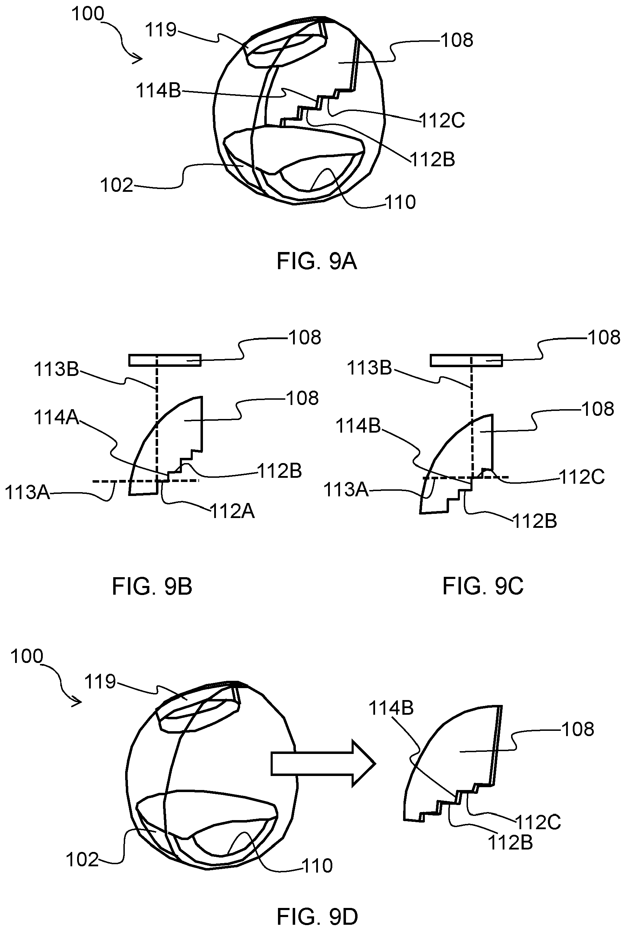

Thus, assembly time may be reduced, e.g. with respect to a scenario in which the structural element(s) 108 is or are distinct component(s) with respect to the container's 102 wall. An example of the latter, which may have cleanability benefits, is nonetheless described herein below with reference to FIG. 9D.

In some embodiments, and with continued reference to FIG. 1, the at least one structural element 108 comprises a ledge portion of the visually transmissive wall 106. Such a ledge portion may be provided at a defined height or heights relative to the milk-supporting base 110, such that milk 104 being observed by the mother to align, e.g. make contact, with the ledge portion is indicative of a given fill level being reached in the container 102.

The at least one structural element 108 may comprise, e.g. be defined by, a visually transmissive element through which milk 104 received in the container 102 is viewable.

In the context of the embodiment shown in FIG. 1, the visually transmissive element is included in the ledge portion of the visually transmissive wall 106.

The visually transmissive element may have a surface 112 contactable by milk 104 when sufficient milk 104 is received in the container 102 to reach the surface 112.

In embodiments, such as shown in FIG. 1, in which the structural element(s) 108 comprise(s) the ledge portion, the surface 112 may comprise, e.g. be defined by, a surface 112 of the ledge portion that is contactable by milk 104 when sufficient milk 104 is received in the container 104 to reach the surface 112 of the ledge portion.

It is noted that the hatching in FIG. 1 represents milk 104 reaching the level of the surface 112 of the ledge portion.

In some embodiments, such as shown in FIG. 1, the structural element(s) 108 is or are arranged at a single elevation, in other words level, relative to the milk-supporting base 110 of the container 102. For example, the surface 112 may be arranged at such a single elevation or level.

In such embodiments, a relatively simple visual estimation of the fill level may be enabled, corresponding to a yes/no assessment of whether or not the milk 104 has reached the elevation/level of the structural element(s) 108, e.g. the elevation/level of the surface 112.

In some embodiments, and referring now to FIGs. 2 and 3, the surface 112; 112A, 112B, 112C comprises surface portions 112A, 112B, 112C arranged at different heights, in other words elevations/levels above the milk-supporting base 110, of the container 102 indicative of different fill levels of milk 104 received in the container 102.

In some embodiments, such as shown in FIG. 2, the surface portions 112A, 112B, 112C may be defined as discrete ledge portion surfaces. This may enable a yes/no assessment of whether or not the milk 104 has reached the elevation/level of each of the surface portions 112A, 112B, 112C.

This may provide the mother with a relatively clear and simple way of tracking filling of the container 102 with milk 104.

It is noted that the hatching in FIG. 2 represents milk 104 reaching the level of the uppermost surface portion 112C, having already previously reached the levels of the lower surface portions 112A, 112B.

In alternative embodiments, such as shown in FIG. 3, the surface 112 comprises a continuously sloping surface 112 along which the surface portions 112A, 112B are provided. Such a continuously sloping surface 112 may provide a continuous measurement of the fill level.

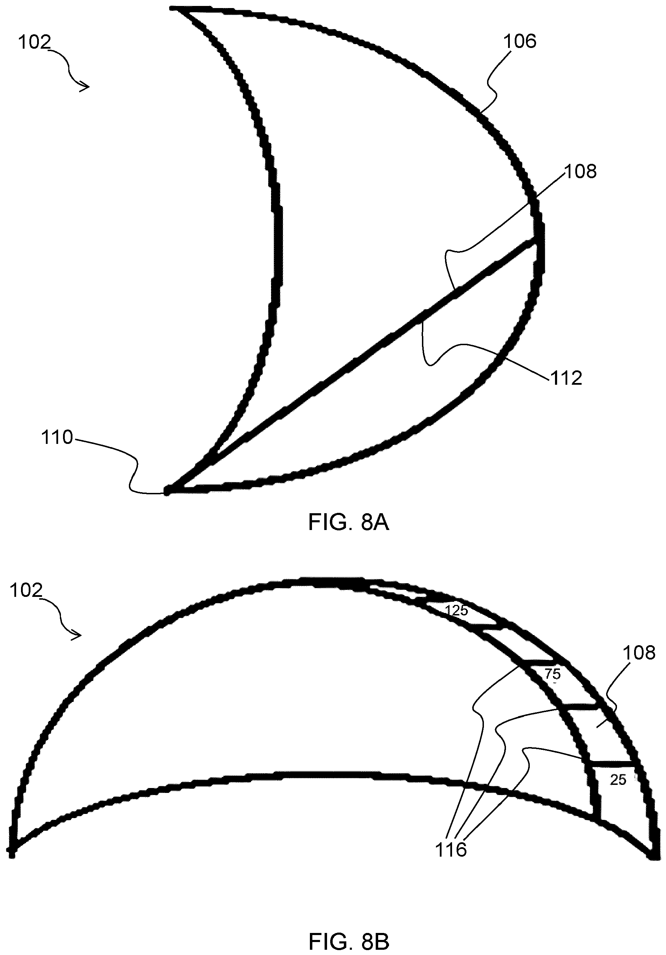

It is noted at this point that fill level markings (not visible in FIGs. 1 to 3) may be provided, e.g. embossed, engraved or printed, on the container 102, for example on the structural element(s) 108. Such fill level markings may, for instance, be provided on the surface 112, e.g. on the continuously sloping surface 112. An example of this is described herein below with reference to FIGs. 8A to 8F.

In embodiments in which the structural element(s) 108 comprise(s) the visually transmissive element, the visually transmissive element may be configured such that light transmitted through the visually transmissive wall 106 is reflected at an interface between the surface 112; 112A, 112B, 112C and air but refracted at an interface between the surface 112; 112A, 112B, 112C and milk 104 in contact with the surface 112; 112A, 112B, 112C. This different reflection/refraction behavior depending on whether or not milk 104 is contacting the surface 112; 112A, 112B, 112C may assist the mother to visually discern whether or not the milk 104 is in contact with the surface 112; 112A, 112B, 112C, or which portion of the surface 112; 112A, 112B, 112C is being contacted by the milk 104.

The refractive index of the visually transmissive element, e.g. made of a suitable visually transmissive plastic material, may be selected to be a sufficiently close match with breast milk 104 that light is refracted at the surface-milk interface.

More generally, the visually transmissive element can be formed from any suitable visually transmissive material, such as polypropylene, e.g. so-called clear polypropylene.

In some embodiments, such as shown in FIGs. 1 to 3, the visually transmissive element is formed from the same material, e.g. polypropylene, as that forming the visually transmissive wall 106.

It is noted that as an alternative to the visually transmissive element, a visually opaque element, e.g. in the form of an opaque plastic member whose position, including elevation relative to the milk-supporting base 110, provides a visual reference against which the level of the milk 104 can be compared by the mother when performing the visual inspection. In embodiments, the visually opaque element comprises one or more areas, with different areas having different colors, wherein different colors provide different context to qualitatively or quantitively interpret a fill level. A fill level reaching an area with a green color may indicate that the container can receive more milk. A fill level reaching an area with a red color may indicate that the milk volume is close to or has reached the maximum capacity of the container.

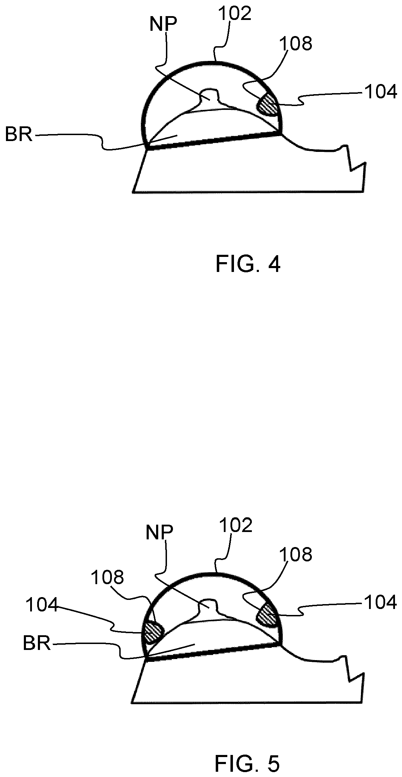

The structural element(s) 108, e.g. the visually transmissive element, may be positioned at any suitable location, for example at a single discrete position, as shown in FIG. 4, or at multiple locations, as shown in FIG. 5.

In embodiments in which the structural element 108, e.g. visually transmissive element, is positioned at a single location, this single location may be such that, during use of the container, it is proximal to, e.g. facing, the mother's other breast, in other words the breast that is not the breast BR on which the container 102 is being worn. An example of this is shown in FIG. 4.

This positioning may facilitate the visual inspection and/or may make performing the visual inspection more discreet because the position proximal to the mother's other breast may be easier to discreetly access in order to perform the visual inspection than a position distal to, e.g. facing away from, the mother's other breast.

In embodiments in which the structural element 108, e.g. visually transmissive element, is positioned at a plurality of locations, such a plurality of locations may include right and left sides of the container 102 when the container 102 is viewed from above. In this way, one of the locations will be proximal, e.g. facing, the mother's other breast, regardless of which breast BR the container 102 is receiving milk 104 from. An example of this is shown in FIG. 5.

Thus, the visual inspection may be facilitated and/or made more discreet when the container 102 is worn on either of the mother's breasts.

In some embodiments, such as shown in FIG. 4 and 5, an exterior surface of the container 102 has one or more recessed portions that each correspond to a bulge of the container's interior surface, with the bulge being included in the at least one structural element 108.

In such embodiments, the recessed portion(s) may assist the mother to grip the container 102, while the corresponding bulge(s) of the container's interior surface may enable the mother to determine the fill level by visual inspection of the bulge(s) and the milk 104 received in the container 102 through the visually transmissive wall 106.

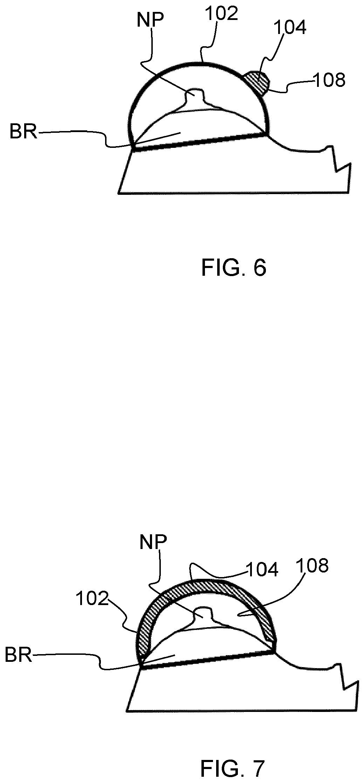

As an alternative or in addition to such recessed portion(s)/bulge(s), the at least one structural element 108 may comprise one or more outwardly protruding portions of the container's exterior surface that each correspond to an indentation in the container's interior surface in which milk 104 is receivable. An example of this is schematically depicted in FIG. 6.

In such embodiments, the outwardly protruding portion(s) may assist with locating and/or orientating the container 102 when the container 102 is being donned by the mother. For example, the outwardly protruding portion(s) may assist the mother to locate and/or orientate the container 102 in the mother's bra. Such functionality of the outwardly protruding portion(s) may be in addition to the indentation(s) in the container's interior surface enabling the mother to determine the fill level by visual inspection of the milk 104 received in the indentation(s) through the visually transmissive wall 106.

As with previous embodiments, the above recesses and protrusions may be positioned to indicate a single fill level or they may be positioned to extend over a range of elevations relative to a milk-supporting base to indicate multiple fill levels.

Whilst FIGs. 4 to 6 depict recessed portion(s)/bulge(s) or outwardly protruding portion(s)/indentation(s) provided at discrete positions, this should not be regarded as being limiting. In some embodiments, such as shown in FIG. 7, a continuous ledge portion extends, when viewed from above, from a righthand side of the container 102 around to a lefthand side of the container 102. A ledge may also be limited to a part of the container, as is shown in FIG. 8B for a sloping ledge. The ledge may be positioned at a side of the container that, during use of the container, faces the other breast of a mother.

FIGs. 8A to 8F each provide side elevation and mother's eye views of a container 102 whose visually transmissive element has a surface 112 contactable by milk 104 when sufficient milk is received in the container 102 to reach the surface 112. The surface 112 comprises a continuously sloping surface 112 along which surface portions at different heights, in other words elevations/levels above the milk-supporting base 110, are provided.

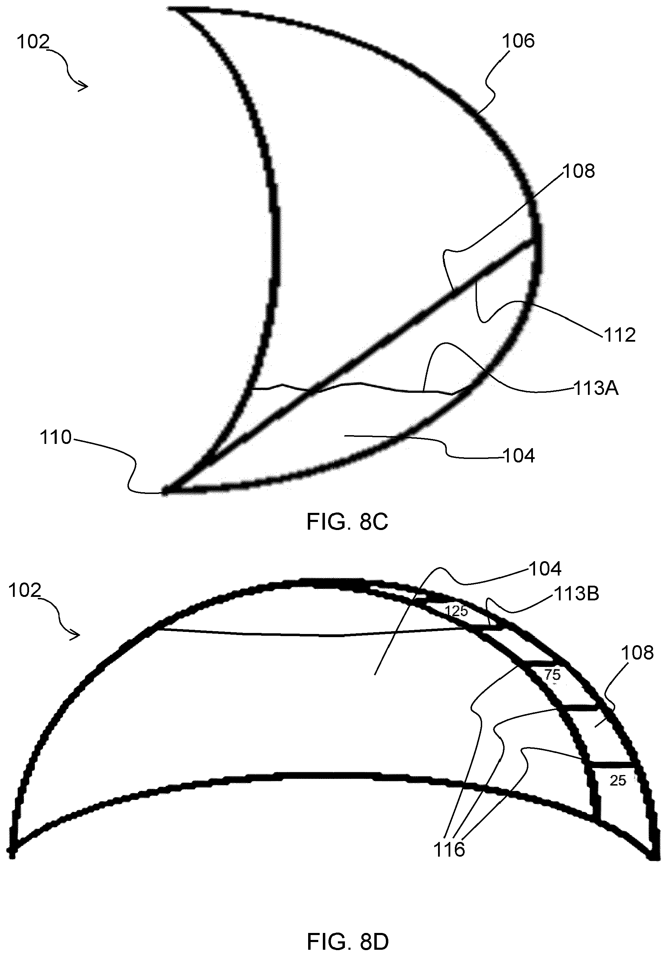

FIGs. 8A and 8B depict the container 102 when no milk 104 is received in the container 102. FIGs. 8C and 8D depict the container 102 when the container 102 is partially filled with milk 104. The milk level 113A above the milk-supporting base 110 of the container 102 is visible from the side elevation provided in FIG. 8C. This milk level 113A corresponds to a position 113B along the continuously sloping surface 112, as shown in FIG. 8D.

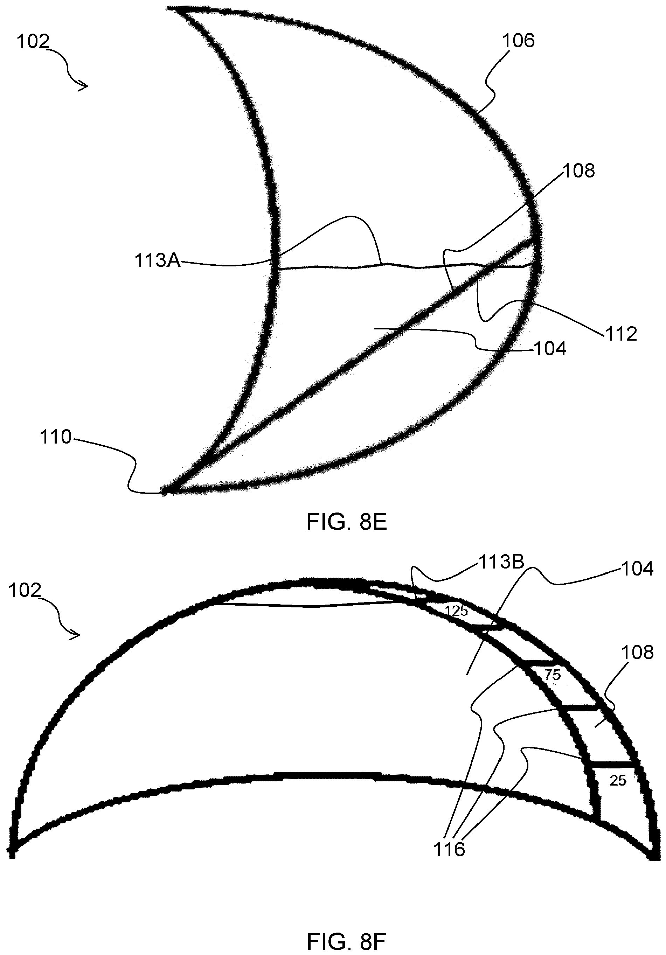

FIGs. 8E and 8F depict the container 102 when substantially filled with milk 104, as evident from the milk level 113A corresponding to a position 113B proximal to the highest portion of the continuously sloping surface 112.

Evident in FIGs. 8A to 8F are fill level markings 116 provided, e.g. embossed, engraved, or printed, on the surface 112. Numerals indicative of the fill level, e.g. in milliliters and/or fluid ounces, may be included in the fill level markings 116.

In the non-limiting example shown in FIGs. 8A to 8F, the fill level markings 116 comprise the numerals 25, 75 and 125, which in this case correspond to numbers of milliliters at different positions along the surface 112.

More generally, a continuous sloping surface-comprising structural element 108 of the type shown in FIGs. 8A to 8F can be regarded as an angled level meter.

Whilst the surface 112 shown in FIGs. 8A, 8C and 8E has a single upward gradient extending from the milk-supporting base 110, so as to provide a linear angled level meter, this is not intended to be limiting and in other embodiments (not visible) a curvedly sloping surface 112 may be employed.

The curvature of such a curvedly sloping surface 112 may, for example, be configured to compensate for the container's 102 interior shape causing non-linearity of milk level with respect to milk fill level. Similarly, discrete structural elements may be positioned such that their appearance appears to change linearly as the fill level rises, when viewed by a mother during use of the container. For example, discrete structural elements may be positioned at equidistant positions around the apparent circumference of the container when viewed from above, while the differences in elevation of the discrete structural elements relative to a milk-supporting base are not of equal size. The apparent positions of the discrete structural elements then compensates for non-linear filling behavior of the container due to its shape.

In some embodiments, such as shown in FIGs. 9A to 9D, the surface 112A, 112B, 112C of the visually transmissive element comprises a stepped surface that includes one or more riser portions 114A, 114B provided between consecutive surface portions 112A, 112B, 112C.

FIG. 9B schematically depicts milk 104 received in the container 102 reaching a level 113A when viewed via the side elevation (lower part of FIG. 9B) that corresponds to a position 113B (upper part of FIG. 9B) on the surface portion 112A.

FIG. 9C schematically depicts more milk 104 received in the container 102, that reaches a level 113A when viewed via the side elevation (lower part of FIG. 9C) that corresponds to a position 113B (upper part of FIG. 9C) on the surface portion 112C.

It is noted that increasing the number of surface portions 112A, 112B, 112C (and riser portions 114A, 114B) to an infinite number would provide a linear diagonal slope of the type depicted in FIGs. 8A to 8F. Lowering the number of surface portions 112A, 112B, 112C (and riser portions 114A, 114B) may enhance robustness of the visual inspection/fill level estimation with respect to tilting of the container 102.

It is reiterated, in the context of the embodiment depicted in FIGs. 9A to 9D, that visual identification of the milk 104 being in contact with the surface 112A, 112B, 112C may be assisted by the visually transmissive element being configured such that light transmitted through the visually transmissive wall 106 is reflected at the interface between the surface 112A, 112B, 112C and air but refracted at the interface between the surface 112A, 112B, 112C and milk 104 in contact with the surface 112A, 112B, 112C.

As noted above, the structural element(s) 108, such as the stepped surface-comprising visually transmissive element shown in FIGs. 9A to 9C, may be an integrated feature of the container 102, in other words may be integrated into the container's 102 wall. However, this is not intended to be limiting and in other embodiments, such as shown in FIG. 9D, the structural element(s) 108, e.g. the visually transmissive element, is or are removable from the container 102.

This removability of the structural element(s) 108, e.g. the visually transmissive element, from the container 102 may make the container 102 and the structural element(s) 108 easier to clean and sterilize.

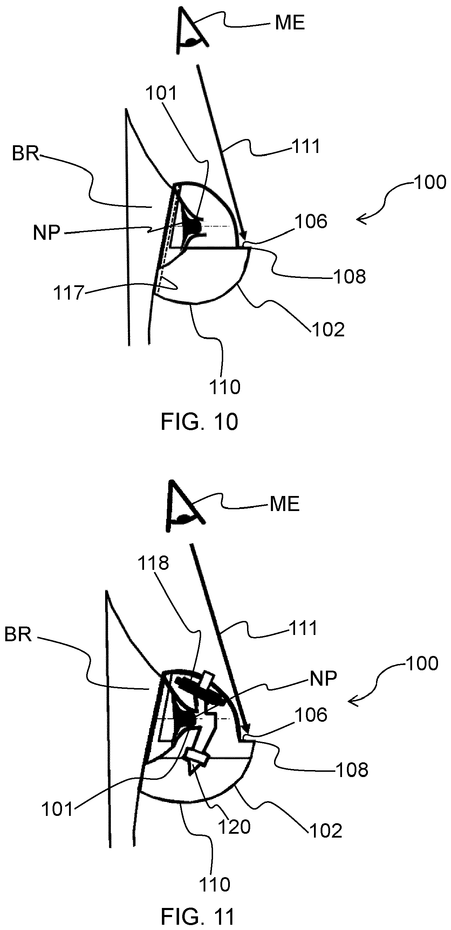

Referring to FIG. 10, the milk expression device 100 comprises the funnel 101 for receiving the nipple-comprising portion NP of the breast BR, and the container 102 for receiving from the funnel 101 milk 104 being expressed by the mother. In such embodiments, the funnel 101 may be included in a first device portion of the milk expression device 100, with the container 102 being included in a second device portion of the milk expression device 100.

These first and second device portions can, for example, be regarded as two "halves" of the milk expression device 100.

In such embodiments, the milk expression device 100 preferably comprises a continuous profile or shape at a point at which the first and second device portions interface with each other.

Such a continuous shape may assist with creating a seal between the first and second device portions.

The continuous seal between the first and second device portions, e.g. "halves", of the milk expression device 100 is denoted in FIG. 10 by the dotted line 117.

In some embodiments, such as shown in FIG. 11, the container 102 and the funnel 101 are detachably coupled to each other. In other words, the container 102 may be separable from the funnel 101, for example by the second device portion being separable from the first device portion. This may facilitate cleaning of the funnel 101 and the container 102.

In some embodiments, the milk expression device 100 includes, as well as the funnel 101 and the container 102, a media separation membrane 118.

Such a media separation membrane 118 may assist to prevent milk 104 from being drawn to a motorized breast pump coupled to, or included in, the milk expression device 100.

Referring again to FIGs. 9A and 9D, the milk expression device 100 may include a holder or chamber 119 in which the media separation membrane 118 is mountable.

In some embodiments, such as shown in FIG. 11, the milk expression device 100 includes a valve 120, such as a one-way valve 120, e.g. a duckbill valve 120.

The valve 120 may assist to prevent milk 104 from flowing back from the container 102 into the funnel 101.

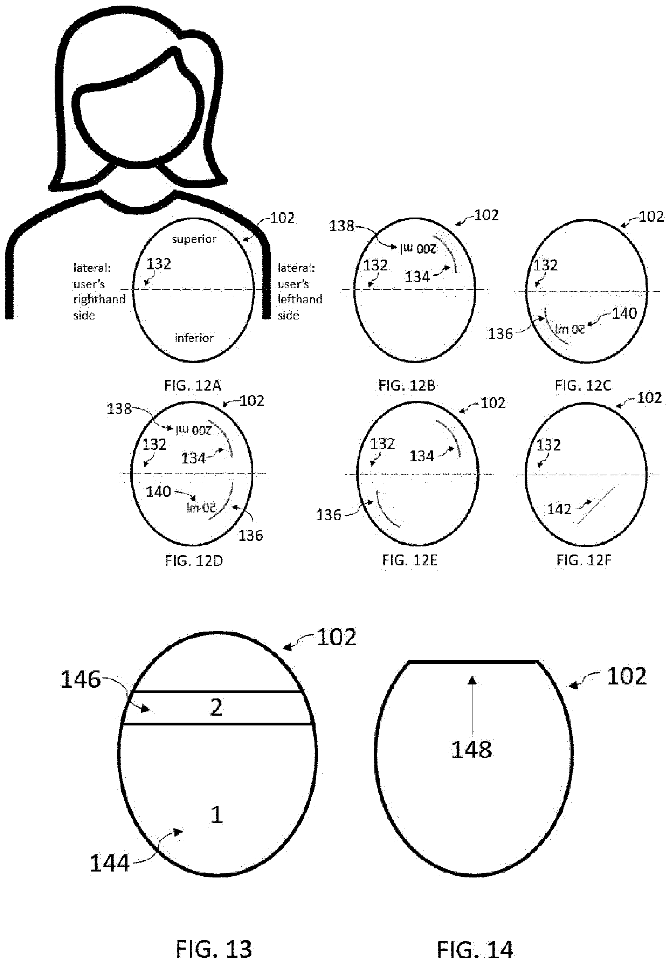

FIGs. 12A-F show schematic frontal views of a container. Here, 'frontal' means `as when facing a container when a mother is using the container'. This is also illustrated in FIG. 12A by the schematic representation of a mother using container 102 on her lefthand breast. FIG. 12A illustrates sides of a container. The container 102 comprises lateral sides corresponding to a user's lefthand and righthand sides. The lateral sides define an area of maximum circumference 132 between them. This area of maximum circumference 132 divides the container 102 in superior and inferior parts. The superior side would be facing a mother using the container during her visual inspection of the fill level of the container during use of the container. The containers shown in FIGs 12A-F may comprise any of the measures presented above to assess the fill level of a container or combinations thereof, including protrusions protruding from either or both of internal and external surfaces of the container and indentations extending into either or both of internal and external surfaces of the container. These measures are not shown in FIGs. 12A-F.

FIG. 12B shows a schematic frontal view of a container according to an example. The superior part comprises fill level markings 134 and 138. Fill level markings 138 represent an alphanumeric or symbolic indication of the volume at a certain fill level. As the fill level markings 138 are comprised in the superior part of container 102, they are upside-down and left-right inverted to make them legible to a mother viewing the superior part during use of the container 102. If, for instance the fill level markings 138 would be a (left-right symmetrical) sad 'smiley', left-right inversion would either not be needed or result in the same symbol, so that the sad smiley only needs to be applied to the superior part upside-down to make it legible to a mother using the container 102.

FIG. 12C shows a schematic frontal view of a container according to an example. The inferior part comprises fill level markings 136 and 140. Fill level markings 136 represent a scale comprising an indication for one or more fill levels. Fill level markings 140 represent an alphanumeric or symbolic indication of the volume at a certain fill level. As the fill level markings 140 are comprised in the inferior part of container 102, they are right-side-up and left-right inverted to make them legible to a mother viewing the inferior part through the superior part and the inner volume of container 102 during use of the container 102. If, for instance the fill level markings 140 would be a (left-right symmetrical) sad 'smiley', left-right inversion would either not be needed or result in the same symbol, so that the sad smiley only needs to be applied to the inferior part right-side-up to make it legible to a mother using the container 102.

FIG. 12D shows a schematic frontal view of a container according to an example. This example is a combination of the examples shown in FIGs. 12B and 12C. the fill level markings 134 and 136 on the one hand, and optionally also the fill level markings 138 and 140 on the other hand, have the same lateral positions on the container 102.

FIG. 12E shows a schematic frontal view of a container according to an example. Fill levels markings 138 and 140 shown in FIGs. 12B-D are optional and are not shown here for simplicity of the figure. In FIG. 12E, fill level makings 134 and 136 have different lateral positions on container 102. This reduces the risk of markings overlapping when a mother performs her visual inspection of the fill level of the container 102 during use of container 102.

FIG. 12F shows a schematic frontal view of a container according to an example. Fill levels markings 140 shown in FIGs. 12C and 12D are optional and are not shown here for simplicity of the figure. Fill level markings 142 extend over a range of lateral positions. This reduces the risk of markings overlapping when a mother performs her visual inspection of the fill level of the container 102 during use of container 102. In FIG. 12F, fill level marking 142 are shown on the inferior side of container 102. However, it will be clear that markings extending laterally may be applied to either or both of the superior and inferior parts of container 102.

Fill level markings 134 and 136 represent a scale comprising an indication for one or more fill levels. The indication may comprise first and second line elements. The first line elements (not explicitly shown in FIGs. 12B-F) may be lines that are parallel to the milk-supporting base. Such first line elements may be distributed along a second line element perpendicular to the first line elements. Fill level markings 134 and 136 may be applied such that, during a mother's visual inspection of the fill level of the container 102 during use of the container 102, they appear to the mother as having been laid out along a straight line. Alternatively or concurrently, the fill level markings 134 and 136 may be applied such that, during a mother's visual inspection of the fill level of the container 102 during use of the container 102, indications of different fill levels (for example the first line elements) appear to the mother as being equidistant to each other. Similar to the explanation given for structural elements, the position of fill level markings may be chosen such that they appear to provide a linear scale to a mother using the container.

The containers shown in FIGs 13-15 may comprise any of the measures presented above to assess the fill level of a container or combinations thereof, including protrusions protruding from either or both of internal and external surfaces of the container and indentations extending into either or both of internal and external surfaces of the container. These measures are not explicitly shown in FIGs. 13-15 for simplicity. The containers shown in FIGs 13-15 may or may also comprise any combination of the fill level markings 134, 136, 138, and 140. These fill level markings may be associated with these measures. For instance, fill level markings comprising an indication of different fill levels may be paired with protrusions out of the external surface of the container at different elevations relative to the milk-supporting base. The fill level markings may comprise or may also comprise characters or symbols assisting in the qualitative or quantitative interpretation of fill levels.

FIG. 13 provides a schematic view of a container according to yet another example. FIG. 13 shows a frontal view just as shown in FIGS. 12A-F. In this example, the container 102 comprises two regions, namely region 1(below the lower horizontal line in FIG. 13) and region 2 (between the two horizontal lines in FIG. 13) indicated by reference numerals 144 and 146 respectively. The two regions are configured such that they present a mother with at least one of a different contrast, a different transparency, and a different color during her visual inspection of the fill level of container 102 during use of the container 102. For instance, region 1 may have a green color indicating that the fill level of the container 102 has not yet reached the level of the maximum capacity for milk. Region 2 may have a red color indicating that the fill level of the container 102 has reached the maximum capacity. The container 102 may comprise any combination of the fill level markings 134, 136, 138, and 140.

FIG. 14 provides a schematic view of a container according to yet another example. The container 102, which may comprise any combination of the fill level markings 134, 136, 138, and 140, comprises a flat viewing surface 148 facing a mother during her visual inspection of the fill level of the container 102 during use of the container 102. The flat viewing surface 148 may be arranged to act as a lens.

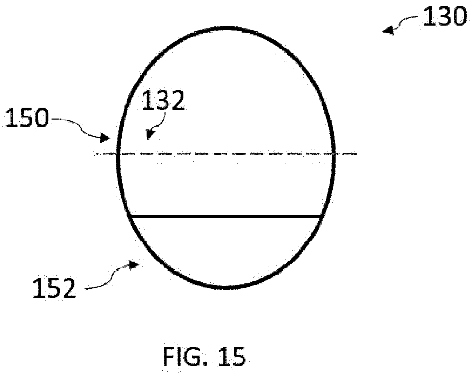

FIG. 15 provides a schematic view of a breast pump comprising a container according to yet another example. The breast pump 130 comprises a first device portion 150 comprising a pump unit. The pump unit may comprise a motor and a battery. The breast pump 130 further comprises a second device portion 152 comprising a container for collecting expressed milk. In FIG. 15, the second device portion 152 is limited to the inferior side of the container 102. The second device portion 152 and, therefore the container, does not extend beyond the area of maximum circumference as defined in FIG. 12A. However, in other examples, the container may extend beyond the area of maximum circumference and occupy at least parts of both the inferior and superior sides of the container 102. In examples, the first device portion 150 and the second device portion 152 are configured to be detachably coupled to each other. In other words, the container may be separable from the funnel, for example by the second device portion 152 being separable from the first device portion 150. This may facilitate cleaning of the funnel and the container. The container 102, may comprise any combination of the fill level markings 134, 136, 138, and 140.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A container (102) for receiving milk (104) being expressed by a mother, the container being at least partly delimited by a visually transmissive wall (106) arranged to be in sight of the mother while the container is being worn by the mother, wherein at least one structural element (108) of the container is arranged, via the at least one structural element's elevation relative to a milk-supporting base (110) of the container, to indicate a fill level by visual inspection by the mother while wearing the container of the at least one structural element and milk received in the container.

2. The container (102) according to claim 1, wherein the at least one structural element (108) comprises a visually transmissive element through which milk received in the container is viewable.

3. The container (102) according to claim 1 or claim 2, wherein the at least one structural element (108) has a surface (112; 112A, 112B, 112C) contactable by milk when sufficient milk is received in the container to reach the surface.

4. The container (102) according to claim 3, wherein the surface (112; 112A, 112B, 112C) comprises surface portions (112A, 112B, 112C) arranged at different heights in the container indicative of different fill levels of milk received in the container.

5. The container (102) according to claim 4, wherein the surface (112; 112A, 112B, 112C) comprises a continuously sloping surface (112) along which said surface portions (112A, 112B) are provided.

6. The container (102) according to claim 4, wherein the surface (112; 112A, 112B, 112C) comprises a stepped surface that includes one or more riser portions (114A, 114B) provided between consecutive surface portions (112A, 112B, 112C).

7. The container (102) according to any one of claims 1 to 6, wherein the at least one structural element (108) is removable from the container.

8. The container (102) according to any one of claims 1 to 7, wherein at least part of the structural element(s) is included in the visually transmissive wall (106).

9. The container (102) according to any one of claims 1 to 8, wherein the at least one structural element (108) comprises a ledge portion of the visually transmissive wall (106).

10. The container (102) according to any one of claims 1 to 9, wherein an exterior surface of the container has one or more recessed portions that each correspond to a bulge of the container's interior surface, said bulge being included in the at least one structural element (108); and/or wherein the at least one structural element comprises one or more outwardly protruding portions of the container's exterior surface that each correspond to an indentation in the container's interior surface in which milk is receivable.

11. The container (102) according to any one of claims 1 to 10, comprising fill level markings (116) paired with the structural element(s) (108).

12. The container (102) according to any one of claims 1 to 11, comprising a background portion for providing a visual contrast with milk (104) that facilitates viewing of the milk.

13. The container (102) according to any one of claims 1 to 12, wherein the container is in the form of a cup receivable in a bra worn by the mother.

14. The container (102) according to any one of claims 1 to 13, wherein at least parts of either or both of the visually transmissive wall (106) and the structural element(s) (108) are not parallel to a mother's line of sight during her visual inspection of the fill level of the container during use of the container.

15. A milk expression device (100) comprising:
a funnel (101) for receiving a nipple-comprising portion of a breast; and
the container (102) according to any one of claims 1 to 14, the container being arranged to receive from the funnel milk being expressed by the mother.
